# EUROPÄISCHE PATENTSCHRIFT

(11) **EP 1 239 818 B1**
(45) Veröffentlichungstag und Bekanntmachung des Hinweises auf die Patenterteilung: **09.06.2004**
(21) Anmeldenummer: 00991979.6
(22) Anmeldetag: 18.12.2000
(51) Int. Cl.: A61K 7/13, A61K 7/06

(54) **ENZYMATISCHES FÄRBEMITTEL**
ENZYMATIC DYE
COLORANT ENZYMATIQUE

(30) Priorität: 24.12.1999 DE 19962882
(43) Veröffentlichungstag der Anmeldung: 18.09.2002
(73) Patentinhaber: Henkel Kommanditgesellschaft auf Aktien, 40589 Düsseldorf-Holthausen (DE)
(72) Erfinder: HÖFFKES, Horst, 40595 Düsseldorf (DE); KLEEN, Astrid, 40699 Erkrath (DE); SÄTTLER, Andrea, 40225 Düsseldorf (DE)
(86) Internationale Anmeldenummer: PCT/EP2000/012894
(87) Internationale Veröffentlichungsnummer: WO 2001/047478

(56) Entgegenhaltungen:
- WO-A-98/56940
- WO-A-99/49010
- WO-A-99/49020

## Beschreibung

Die vorliegende Erfindung betrifft Mittel zum Färben keratinischer Fasern, die mindestens ein Phenol-oxidierendes Enzym, das aus *Stachybotrys Spezies* gewonnen werden kann, enthalten, entsprechende Verfahren zum Färben keratinischer Fasern sowie die Verwendung eines Phenol-oxidierenden Enzyms, das aus *Stachybotrys Spezies* gewonnen werden kann, zur oxidativen Haarfärbung.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muß üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Die oxidative Entwicklung der Färbung kann grundsätzlich mit Luftsauerstoff erfolgen. Bevorzugt wird jedoch ein chemisches Oxidationsmittel eingesetzt. Als Oxidationsmittel kommen Persulfate, Chlorite und insbesondere Wasserstoffperoxid oder dessen Anlagerungsprodukte an Harnstoff, Melamin sowie Natriumborat in Frage. Üblicherweise wird eine etwa 2-9%ige wäßrige Wasserstoffperoxidlösung eingesetzt. Unter der Einwirkung derart hoher Oxidationsmittelkonzentrationen können die keratinischen Fasern, insbesondere wenn sie bereits dauergewellt oder gebleicht sind, geschädigt werden, und in seltenen Fällen können durch diese hohen Konzentrationen auch Hautirritationen auftreten.

Ein wesentlicher Lösungsansatz zu dieser Problematik geht von der Reduzierung der Oxidationsmittelkonzentration aus. Es wurde daher in der Vergangenheit einerseits nach Farbstoffvorprodukten gesucht, die aufgrund ihrer chemischen Struktur bereits durch geringere Mengen Wasserstoffperoxid oder durch Luftsauerstoff oxidiert werden können. Andererseits wurde die Verwendung von Enzymen als Biokatalysatoren vorgeschlagen, die den erwünschten Oxidationsprozess mit sehr wenig oder ganz ohne Wasserstoffperoxid nur in der Anwesenheit von Luftsauerstoff katalysieren können.

In der deutschen Offenlegungsschrift DE-OS-21 55 390 wird ein enzymaktiviertes, oxidatives Haarfärbeverfahren beschrieben, bei dem geringe Mengen H₂O₂ in Kombination mit einer Peroxidase eingesetzt werden. Auch in der EP-A1-0 310 675 werden enzymatische Haarbehandlungsmittel offenbart, die mindestens eine Dielektronen-reduzierende Oxidase, die Sauerstoff als Akzeptor nutzt, enthalten. In der EP-B1-0 548 620 werden enzymatische Haarfärbemittel beschrieben, bei denen die Oxidation der Farbstoffvorprodukte durch Einsatz einer Peroxidase katalysiert wird. Schließlich werden in der EP-A2-0 795 313 enzymatische Haarfärbemittel beschrieben, die ein Sauerstoff-Oxidoreduktase/Substrat-System und eine Peroxidase sowie als Kupplerkomponenten zwingend ein m-Phenylendiaminderivat enthalten. Alle diese Färbemittel können aber bezüglich der erzielbaren Färbeleistung (Intensität, Nuance, Glanz, Echtheitseigenschaften) noch nicht vollständig überzeugen.

Die Technik der leicht-oxidierbaren Farbstoffvorprodukte hat ebenso wie die bislang beschriebene enzymatische Farbentwicklung den Nachteil, daß im Vergleich zu den herkömmlichen Verfahren insbesondere bezüglich der Intensität, des Glanzes und der Echtheitseigenschaften der Färbungen schlechtere Ergebnisse erzielt werden.

Die vorliegende Erfindung geht daher von der Aufgabe aus, Färbemittel für keratinische Fasern zur Verfügung zu stellen, die eine schonende Faserbehandlung ermöglichen und gleichzeitig eine hervorragende Färbeleistung gewährleisten.

Ein erster Gegenstand der vorliegenden Erfindung sind daher Mittel zum Färben keratinischer Fasern, die in einem kosmetisch akzeptablen Träger mindestens ein Farbstoffvorprodukt sowie mindestens ein Phenol-oxidierendes Enzym, das aus *Stachybotrys Spezies* gewonnen werden kann, enthalten.

Unter keratinischen Fasern sind erfindungsgemäß Pelze, Wolle, Federn und insbesondere menschliche Haare zu verstehen.

Erfindungsgemäß bevorzugt sind Phenol-oxidierende Enzyme, die aus *Stachybotrys Spezies* gewonnen werden können, wie sie in den internationalen Offenlegungsschriften WO-A2-99/49020 und WO-A2-99/49010 beschrieben werden. Insbesondere auf die Offenbarung zur Gewinnung und Reinigung dieser Enzyme der WO-A2-99/49020 wird an dieser Stelle explizit Bezug genommen. Erfindungsgemäß sind gereinigte Enzymzubereitungen bevorzugt. Beispiele für erfindungsgemäß einsetzbare *Stachybotrys Spezies* sind *Stachybotrys parvispora*, *Stachybotrys chartarum*, *Stachybotrys kampalensis*, *Stachybotrys theobromae*, *Stachybotrys bisbyi*, *Stachybotrys cylindrospora*, *Stachybotrys dichroa*, *Stachybotrys oenanthes* und *Stachybotrys nilagerica*. Auch bezüglich der Derivatisierung und der Anwendungsformen dieser Enzyme wird ausdrücklich auf die Offenbarung der oben genannten internationalen Offenlegungsschriften Bezug genommen.

Das Enzym kann in das Färbemittel selbst eingearbeitet werden; bevorzugt wird es aber getrennt von den Farbstoffvorprodukten konfektioniert und erst unmittelbar vor der Anwendung dem Färbemittel zugegeben.

Das Enzym wird bevorzugt in einer Menge von 0,001 - 1 Gew.-%, bezogen auf die Proteinmenge des Enzyms und das gesamte Färbemittel, eingesetzt.

Hinsichtlich der in den erfindungsgemäßen Färbemitteln eingesetzten Farbstoffvorprodukte unterliegt die vorliegende Erfindung keinerlei Einschränkungen. Die erfindungsgemäßen Färbemittel können als Farbstoffvorprodukte
- Oxidationsfarbstoffvorprodukte vom Entwickler- und/oder Kuppler-Typ, und
- Vorstufen naturanaloger Farbstoffe, wie Indol- und Indolin-Derivate,
sowie Mischungen von Vertretern dieser Gruppen enthalten.

Als Entwicklerkomponenten werden üblicherweise primäre aromatische Amine mit einer weiteren, in para- oder ortho-Position befindlichen, freien oder substituierten Hydroxyoder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (I) 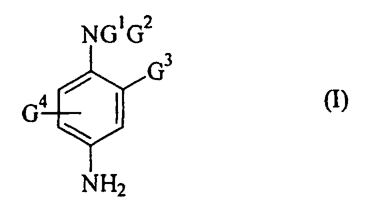 wobei
- G¹ steht für ein Wasserstoffatom, ein C₁- bis C₄-Alkylradikal, ein C₁- bis C₄-Monohydroxyalkylradikal, ein C₂- bis C₄-Polyhydroxyalkylradikal, ein (C₁- bis C₄)-Alkoxy-(C₁- bis C₄)-alkylradikal, ein 4'-Aminophenylradikal oder ein C₁- bis C₄-Alkylradikal, das mit einer stickstoffhaltigen Gruppe, einem Phenyl- oder einem 4'-Aminophenylrest substituiert ist;
- G² steht für ein Wasserstoffatom, ein C₁- bis C₄-Alkylradikal, ein C₁- bis C₄-Monohydroxyalkylradikal, ein C₂- bis C₄-Polyhydroxyalkylradikal, ein (C₁- bis C₄)-Alkoxy-(C₁- bis C₄)-alkylradikal oder ein C₁- bis C₄-Alkylradikal, das mit einer stickstoffhaltigen Gruppe substituiert ist;
- G³ steht für ein Wasserstoffatom, ein Halogenatom, wie ein Chlor-, Brom, Jododer Fluoratom, ein C₁- bis C₄-Alkylradikal, ein C₁- bis C₄-Monohydroxyalkylradikal, ein C₁- bis C₄-Hydroxyalkoxyradikal, ein C₁- bis C₄-Acetylaminoalkoxyradikal, ein C₁- bis C₄- Mesylaminoalkoxyradikal oder ein C₁- bis C₄-Carbamoylaminoalkoxyradikal;
- G⁴ steht für ein Wasserstoffatom, ein Halogenatom oder ein C₁- bis C₄-Alkylradikal oder
- wenn G³ und G⁴ in ortho-Stellung zueinander stehen, können sie gemeinsam eine verbrückende α,ω-Alkylendioxogruppe, wie beispielsweise einen Ethylendioxygruppe bilden.

Beispiele für die als Substituenten in den erfindungsgemäßen Verbindungen genannten, C₁- bis C₄-Alkylradikale sind die Gruppen Methyl, Ethyl, Propyl, Isopropyl und Butyl. Ethyl und Methyl sind bevorzugte Alkylradikale. Erfindungsgemäß bevorzugte C₁- bis C₄-Alkoxyradikale sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyalkylgruppe ist besonders bevorzugt. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (II) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (I) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-pphenylendiamin, N,N-Dipropyl-p-phenylendiamin, 4-Amino-3-methyl-(N,N-diethyl)-anilin, N,N-bis-(β-Hydroxyethyl)-p-phenylendiamin, 4-N,N-bis-(β-Hydroxyethyl)amino-2-methylanilin, 4-N,N-bis-(β-Hydroxyethyl)amino-2-chloranilin, 2-(β-Hydroxyethyl)-pphenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,-β-hydroxyethyl)-p-phenylendiamin, N-(β,γ-Dihydroxypropyl)-p-phenylendiamin, N-(4'-Aminophenyl)-p-phenylendiamin, N-Phenylp-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (I) sind p-Phenylendiamin, p-Toluylendiamin, 2-(β-Hydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Aminound/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (II) entsprechen, sowie ihre physiologisch verträglichen Salze: 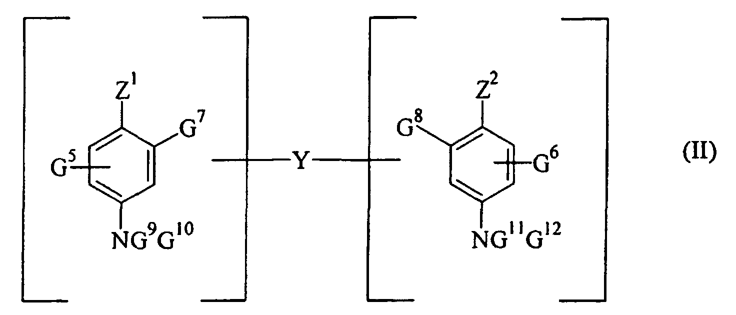 wobei:
- Z¹ und Z² stehen unabhängig voneinander für ein Hydroxyl- oder NH₂-Radikal, das
- gegebenenfalls durch ein C₁- bis C₄-Alkylradikal, durch ein C₁- bis C₄-Hydroxyalkylradikal und/oder durch eine Verbrückung Y substituiert ist,
- die Verbrückung Y steht für eine Alkylengruppe mit 1 bis 14 Kohlenstoffatomen,
- wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch ein oder mehrere Hydroxyl- oder C₁- bis C₈-Alkoxyradikale substituiert sein kann,
- G⁵ und G⁶ stehen unabhängig voneinander für ein Wasserstoff- oder Halogenatom,
- ein C₁- bis C₄-Alkylradikal, ein C₁- bis C₄-Monohydroxyalkylradikal, ein C₂- bis C₄- Polyhydroxyalkylradikal, ein C₁- bis C₄-Aminoalkylradikal oder eine direkte Verbindung zur Verbrückung Y,
- G⁷, G⁸, G⁹, G¹⁰, G¹¹ und G¹² stehen unabhängig voneinander für ein Wasserstoff-
- atom, eine direkte Bindung zur Verbrückung Y oder ein C₁- bis C₄-Alkylradikal, mit der Maßgabe, daß die Verbindungen der Formel (II) nur eine Verbrückung Y pro Molekül enthalten.

Die in Formel (II) verwendeten Substituenten sind erfindungsgemäß analog zu den obigen Ausführungen definiert.

Bevorzugte zweikemige Entwicklerkomponenten der Formel (II) sind insbesondere: N,N'-bis-(β-Hydroxyethyl)-N,N'-bis-(4'-Aminophenyl)-1,3-diamino-propanol, N,N'-bis-(β-Hydroxyethyl)-N,N'-bis-(4'-aminophenyl)-ethylendiamin, N,N'-bis-(4-Aminophenyl)-tetramethylendiamin, N,N'-bis-(β-Hydroxyethyl)-N,N'-bis-(4-aminophenyl)-tetramethylendiamin, N,N'-bis-(4-Methyl-aminophenyl)-tetramethylendiamin, N,N'-bis-(Ethyl)-N,N'-bis(4'-amino,3'-methylphenyl)-ethylendiamin, 1,8-bis-(2,5-Diaminophenoxy)-3,5-dioxaoktan, Bis-(2-hydroxy-5-aminophenyl)-methan, 1,4-Bis-(4-aminophenyl)-diaza-cycloheptan und 1,10-Bis-(2,5-diaminophenyl)-1,4,7,10-tetraoxadecan und ihre physiologisch verträglichen Salze.

Ganz besonders bevorzugte zweikernige Entwicklerkomponenten der Formel (II) sind N,N'-bis-(β-Hydroxyethyl)-N,N'-bis-(4'-aminophenyl)-1,3-diamino-propanol, Bis-(2-hydroxy-5-aminophenyl)-methan, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (III) 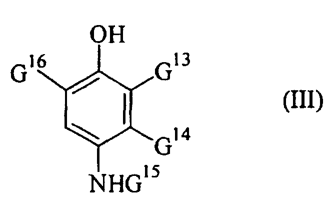 wobei:
- G¹³ steht für ein Wasserstoffatom, ein Halogenatom, ein C₁- bis C₄-Alkylradikal, ein C₁- bis C₄-Monohydroxyalkylradikal, ein (C₁- bis C₄)-Alkoxy-(C₁- bis C₄)-alkylradikal, ein C₁- bis C₄-Aminoalkylradikal, ein Hydroxy-(C₁- bis C₄)-alkylaminoradikal, ein C₁- bis C₄-Hydroxyalkoxyradikal, ein C₁- bis C₄-Hydroxyalkyl-(C₁-bis C₄)-aminoalkylradikal oder ein (Di-C₁- bis C₄-Alkylamino)-(C₁- bis C₄)-alkylradikal, und
- G¹⁴ steht für ein Wasserstoff- oder Halogenatom, ein C₁- bis C₄-Alkylradikal, ein C₁- bis C₄-Monohydroxyalkylradikal, ein C₂- bis C₄-Polyhydroxyalkylradikal, ein (C₁- bis C₄)-Alkoxy-(C₁- bis C₄)-alkylradikal, ein C₁- bis C₄-Aminoalkylradikal oder ein C₁- bis C₄-Cyanoalkylradikal,
- G¹⁵ steht für Wasserstoff, ein C₁- bis C₄-Alkylradikal, ein C₁- bis C₄-Monohydroxyalkylradikal, ein C₂- bis C₄-Polyhydroxyalkylradikal, ein Phenylradikal oder ein Benzylradikal, und
- G¹⁶ steht für Wasserstoff oder ein Halogenatom.

Die in Formel (III) verwendeten Substituenten sind erfindungsgemäß analog zu den obigen Ausführungen definiert.

Bevorzugte p-Aminophenole der Formel (III) sind insbesondere p-Aminophenol, N-Methyl-p-Aminophenol, 4-Amino-3-methyl-phenol, 4-Amino-3-fluorphenol, 2-Hydroxymethylamino-4-amino-phenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethylaminomethyl)-phenol, 4-Amino-2-fluorophenol, 4-Amino-2-chlorphenol, 2,6-Dichlor-4-aminophenol, 4-Amino-2-((diethylamino)methyl)phenol sowie ihre physiologisch verträglichen Salze.

Ganz besonders bevorzugte Verbindungen der Formel (III) sind p-Aminophenol, 4-Amino-3-methylphenol, 4-Amino-2-chlorphenol, 4-Amino-2,6-dichlorphenol, 4-Amino-2-aminomethylphenol und 4-Amino-2-((diethylamino)methyl)phenol.

Ferner kann die Entwicklerkomponente ausgewählt sein aus o-Aminophenol und seinen Derivaten, wie beispielsweise 2-Amino-4-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere die Verbindungen, die im deutschen Patent DE 23 59 399, der japanischen Offenlegungsschrift JP-A2-02/019576 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 38 43 892, DE 41 33 957 und Offenlegungsschriften WO 94/08969, WO 94/08970, EP 0 740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorobenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2'aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4(β-hydroxyethyl)amino-1-methylpyrazol.

Bevorzugte Pyrazol-Pyrimidin-Derivate sind insbesondere die Derivate des Pyrazol-[1,5-a]-pyrimidin der folgenden Formel (IV) und dessen tautomeren Formen, sofern ein tautomerisches Gleichgewicht besteht: 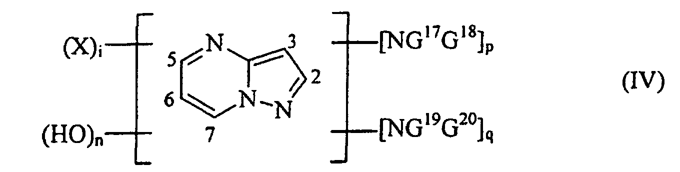 wobei:
- G¹⁷, G¹⁸, G¹⁹ und G²⁰ unabhängig voneinander stehen für ein Wasserstoffatom, ein
- C₁- bis C₄-Alkylradikal, ein Aryl-Radikal, ein C₁- bis C₄-Hydroxyalkylradikal, ein C₂- bis C₄-Polyhydroxyalkylradikal ein (C₁- bis C₄)-Alkoxy-(C₁- bis C₄) -alkylradikal, ein C₁- bis C₄-Aminoalkylradikal, das gegebenenfalls durch ein Acetyl-Ureid- oder Sulfonyl-Radikal geschützt sein kann, ein (C₁- bis C₄) -Alkylamino-(C₁- bis C₄)-alkylradikal, ein Di-[(C₁- bis C₄)-alkyl]-(C₁- bis C₄) -aminoalkylradikal, wobei die Dialkyl-Radikale gegebenenfalls einen Kohlenstoffzyklus oder einen Heterozyklus mit 5 oder 6 Kettengliedern bilden, ein C₁- bis C₄-Hydroxyalkyl- oder ein Di-(C₁- bis C₄)-[Hydroxyalkyl]-(C₁- bis C₄)-aminoalkylradikal,
- die X-Radikale stehen unabhängig voneinander für ein Wasserstoffatom, ein C₁bis
   C₄-Alkylradikal, ein Aryl-Radikal, ein C₁- bis C₄-Hydroxyalkyladikal, ein C₂- bis C₄- Polyhydroxyalkylradikal, ein C₁- bis C₄-Aminoalkylradikal, ein (C₁- bis C₄) -Alkylamino-(C₁- bis C₄)-alkylradikal, ein Di-[(C₁- bis C₄)alkyl]- (C₁- bis C₄) -aminoalkylradikal, wobei die Dialkyl-Radikale gegebenenfalls einen Kohlenstoffzyklus oder einen Heterozyklus mit 5 oder 6 Kettengliedern bilden, ein C₁- bis C₄-Hydroxyalkyl- oder ein Di-(C₁- bis C₄-hydroxyalkyl)-aminoalkylradikal, ein Aminoradikal, ein C₁- bis C₄-Alkyl- oder Di-(C₁- bis C₄hydroxyalkyl)-aminoradikal, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, daß
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (IV) verwendeten Substituenten sind erfindungsgemäß analog zu den obigen Ausführungen definiert.

Wenn das Pyrazol-[1,5-a]-pyrimidin der obenstehenden Formel (IV) eine Hydroxygruppe an einer der Positionen 2, 5 oder 7 des Ringsystems enthält, besteht ein tautomeres Gleichgewicht, das zum Beispiel im folgenden Schema dargestellt wird:

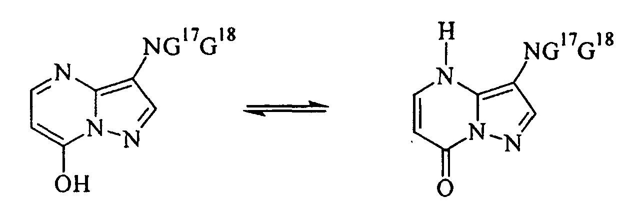

Unter den Pyrazol-[1,5-a]-pyrimidinen der obenstehenden Formel (IV) kann man insbesondere nennen:
- Pyrazol-[1,5-a]-pyrimidin-3,7-diamin;
- 2,5-Dimethyl pyrazol-[1,5-a]-pyrimidin-3,7-diamin;
- Pyrazol-(1,5-a]-pyrimidin-3,5-diamin;
- 2,7-Dimethyl pyrazol-[1,5-a]-pyrimidin-3,5-diamin;
- 3-Amino pyrazol-[1,5-a]-pyrimidin-7-ol;
- 3-Amino pyrazol-[1,5-a]-pyrimidin-5-ol;
- 2-(3-Amino pyrazol-[1,5-a]-pyrimidin-7-ylamino)-ethanol;
- 2-(7-Amino pyrazol-[1,5-a]-pyrimidin-3-ylamino)-ethanol;
- 2-[(3-Amino pyrazol-[1,5-a]-pyrimidin-7-yl)-(2-hydroxy-ethyl)-amino]-ethanol;
- 2-[(7-Amino pyrazol-[1,5-a]-pyrimidin-3-yl)-(2-hydroxy-ethyl)-amino]-ethanol;
- 5,6-Dimethyl pyrazol-[1,5-a]-pyrimidin-3,7-diamin;
- 2,6-Dimethyl pyrazol-[1,5-a]-pyrimidin-3,7-diamin;
- 2,5, N7, N7-Tetramethyl pyrazol-[1,5-a]-pyrimidin-3,7-diamin;
sowie ihre physiologisch verträglichen Salze und ihre tautomeren Formen, wenn ein tautomerisches Gleichgewicht vorhanden ist.

Die Pyrazol-[1,5-a]-pyrimidine der obenstehenden Formel (IV) können wie in der Literatur beschrieben durch Zyklisierung ausgehend von einem Aminopyrazol oder von Hydrazin hergestellt werden.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind:
- m-Aminophenol und dessen Derivate wie beispielsweise 5-Amino-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol,

Besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die Oxidationsfarbstoffvorprodukte sind in den erfindungsgemäßen Mitteln bevorzugt in Mengen von 0,01 bis 20 Gew.-%, vorzugsweise 0,5 bis 5 Gew.-%, jeweils bezogen auf das gesamte Mittel, enthalten.

Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe.

Besonders gut als Vorstufen naturanaloger Haarfarbstoffe geeignet sind Derivate des 5,6-Dihydroxyindolins der Formel (Va), 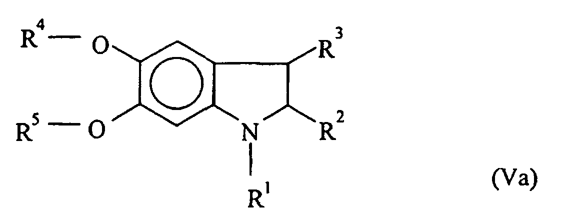 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (Vb), 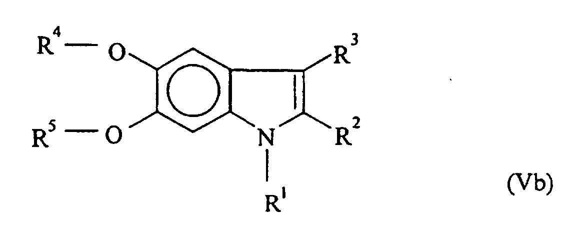 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.
Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolinoder Indolderivat in Haarfärbemitteln in Kombination mit mindestens einer Aminosäure oder einem Oligopeptid einzusetzen. Die Aminosäure ist vorteilhafterweise eine α-Aminosäure ist; ganz besonders bevorzugte α-Aminosäuren sind Arginin, Omithin, Lysin und Histidin, insbesondere Arginin.

Neben den Farbstoffvorprodukten können die erfindungsgemäßen Färbemittel zur weiteren Nuancierung direktziehende Farbstoffe enthalten. Diese sind üblicherweise ausgewählt aus Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, HC Orange 1, Disperse Orange 3, HC Red 1, HC Red 3, HC Red 13, HC Red BN, Basic Red 76, HC Blue 2, HC Blue 12, Disperse Blue 3, Basic Blue 7, Basic Blue 26, Basic Blue 99, HC Violet 1, Disperse Violet 1, Disperse Violet 4, Basic Violet 2, Basic Violet 14, Acid Violet 43, Disperse Black 9, Acid Black 52, Basic Brown 16 und Basic Brown 17 bekannten Verbindungen sowie 1,4-Bis-(β-hydroxyethyl)-amino-2-nitrobenzol, 3-Nitro-4-(β-hydroxyethyl)-aminophenol, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Neben dem erfindungsgemäßen Oxidasesystem können die erfindungsgemäßen Färbemittel auch weitere Enzyme, wie beispielsweise Laccasen, Peroxidasen doer Oxidasen mit ihren jeweiligen Substraten enthalten. Bevorzugte Oxidasen sind beispielsweise Cholin-Oxidase, Glucose-Oxidase, Alkohol-Oxidase, Pyruvat-Oxidase, Oxalat-Oxidase, Cholesterin-Oxidase, Uricase, Lactat-Oxidase, Xanthin-Oxidase, Pyranose-Oxidase, Glycerin-Oxidase sowie Galactose-Oxidase. Im Rahmen dieser Ausführungsform besonders bevorzugt sind Mittel, die neben dem Phenol-oxidierenden Enzym weiterhin Uricase, Glucose-Oxidase und/oder Xanthin-Oxidase sowie deren jeweilige Substrate enthalten.

Zur Herstellung der erfindungsgemäßen Färbemittel können die Farbstoffvorprodukte in einen geeigneten wasserhaltigen Träger eingearbeitet werden. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, die für die Anwendung auf dem Haar geeignet sind.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. Der Fachmann kann einen eventuellen Einfluß der verschiedenen Tenside auf die Aktivität des erfindungsgemäßen Enzymsystems gegebenenfalls durch einfache Vorversuche überprüfen.

In einer bevorzugten Ausführungsform der vorliegenden Erfindung wird in den Mitteln zur Färbung keratinischer Fasern eine Kombination aus anionischen und nichtionischen Tensiden oder eine Kombination aus anionischen und amphoteren Tensiden eingesetzt. Es hat sich aber in Einzelfällen als vorteilhaft erwiesen, die Tenside aus amphoteren oder nichtionischen Tensiden auszuwählen, da diese in der Regel den erfindungsgemäßen Färbeprozeß weniger beeinflussen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)_{X}. Diese Verbindungen sind beispielsweise unter dem Handelsnamen Plantacare® von Henkel erhältlich und sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen. Ein erfindungsgemäß besonders bevorzugtes Alkylglucosid wird ist das Handelsprodukt Plantacare® 1200G.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.
Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quatemium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quatemierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quatemium-80).

Ein Beispiel für ein als kationisches Tensid einsetzbares quatemäres Zuckerderivat stellt das Handelsprodukt Glucquat®100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin enthalten die erfindungsgemäßen Mittel bevorzugt mindestens ein Alkalisierungsmittel. Bevorzugte Alkalisierungsmittel sind Ammoniak, Monoethanolamin, Natriumhydroxid, Kaliumhydroxid und insbesondere Arginin, Lysin und Histidin.

Weiterhin können die erfindungsgemäßen Färbemittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten. Hinsichtlich der kationische Tenside sei auf die obigen Ausführungen verwiesen.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quatemisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate.
- polymere Dimethyldiallylammoniumsalze und deren Copolymere mit Acrylsäure sowie Estern und Amiden von Acrylsäure und Methacrylsäure. Die unter den Bezeichnungen Merquat®100 (Poly(dimethyldiallylammoniumchlorid)), Merquat®550 (Dimethyldiallylammoniumchlorid-Acrylamid-Copolymer) und Merquat® 280 (Dimethyldiallylammoniumchlorid-Acrylsäure-Copolymer im Handel erhältlichen Produkte sind Beispiele für solche kationischen Polymere.
- Copolymere des Vinylpyrrolidons mit quatemierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat®734 und Gafquat®755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen sowie Polyquaternium-2, ganz besonders bevorzugt sind Polyquaternium-2, Polyquaternium-10 und Polyquaternium-22. Unter den als Polyquaternium-2 bekannten Verbindungen wird insbesondere das Handelprodukt Mirapol®A-15 bevorzugt.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weiterhin enthalten die erfindungsgemäß verwendeten Zubereitungen bevorzugt mindestens eine Ölkomponente.

Erfindungsgemäß geeignete Ölkomponenten sind prinzipiell alle wasserunlöslichen Öle und Fettstoffe sowie deren Mischungen mit festen Paraffinen und Wachsen. Als wasserunlöslich werden erfindungsgemäß solche Stoffe definiert, deren Löslichkeit in Wasser bei 20 °C kleiner als 0,1 Gew.-% beträgt. Der Schmelzpunkt der einzelnen Öloder Fettkomponenten liegt bevorzugt unterhalb von etwa 40 °C. Öl- und Fettkomponenten, die bei Raumtemperatur, d. h. unterhalb von 25 °C flüssig sind, können erfindungsgemäß besonders bevorzugt sein. Bei Verwendung mehrerer Öl- und Fettkomponenten sowie ggf. festen Paraffinen und Wachsen ist es in der Regel jedoch auch ausreichend, wenn die Mischung der Öl- und Fettkomponenten sowie ggf. Paraffine und Wachse diesen Bedingungen genügt.

Eine bevorzugte Gruppe von Ölkomponenten sind pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls.

Geeignet sind aber auch andere Triglyceridöle wie die flüssigen Anteile des Rindertalgs sowie synthetische Triglyceridöle.

Eine weitere, besonders bevorzugte Gruppe erfindungsgemäß als Ölkomponente einsetzbarer Verbindungen sind flüssige Paraffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-nundecylether, Di-n-dodecylether, n-Hexyl-n-octylether, n-Octyl-n-decylether, n-Decyl-nundecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-noctylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.

Ebenfalls erfindungsgemäß einsetzbare Ölkomponenten sind Fettsäure- und Fettalkoholester. Bevorzugt sind die Monoester der Fettsäuren mit Alkoholen mit 3 bis 24 C-Atomen. Bei dieser Stoffgruppe handelt es sich um die Produkte der Veresterung von Fettsäuren mit 6 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen, bei der Oxidation von Aldehyden aus der Roelen'schen Oxosynthese oder der Dimerisierung von ungesättigten Fettsäuren anfallen, mit Alkoholen wie beispielsweise Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat, Isononansäure-C16-18-alkylester (Cetiol® SN), Stearinsäure-2-ethylhexylester (Cetiol® 868), Cetyloleat, Glycerintricaprylat, Kokosfettalkohol-caprinat/-caprylat und n-Butylstearat.

Weiterhin stellen auch Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-diisostearat und Neopentylglykoldi-capylat erfindungsgemäß verwendbare Ölkomponenten dar, ebenso komplexe Ester wie z. B. das Diacetyl- glycerinmonostearat.

Schließlich können auch Fettalkohole mit 8 bis 22 C-Atomen als erfindungsgemäß wirkende Ölkomponenten eingesetzt werden. Die Fettalkohole können gesättigt oder ungesättigt und linear oder verzweigt sein. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen.

Die Ölkomponenten werden bevorzugt in Menden von 0,05 bis 10 Gew.-%, insbesondere von 0,1 bis 2 Gew.-% in den erfindungsgemäßen Färbemitteln eingesetzt.

In einer bevorzugten Ausführungsform der vorliegenden Erfindung bildet sich bei Auflösung der Färbemittel in Wasser ein Gel. Hierzu werden dem Färbemittel Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol zugesetzt. Besonders bevorzugte Verdickungsmittel sind Xanthane, Alginate sowie hochsubstituierte Carboxymethylcellulosen.

Weitere Wirk-, Hilfs- und Zusatzstoffe sind beispielsweise
- zwitterionische und amphotere Polymere wie beispielsweise Acrylamidopropyl-trimethylammoniumchlorid/Acrylat-Copolymere und Octylacrylamid/Methylmethacrylat/tert-Butylaminoethylmethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Strukturanten wie Maleinsäure und Milchsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Zweckmäßigerweise wird die Enzymzubereitung unmittelbar vor dem Haarefärben mit der Zubereitung aus den Farbstoffvorprodukten vermischt. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

In einer weiteren Ausführungsform der vorliegenden Erfindung kann es bevorzugt sein, die Enzymzubereitung frei von Antioxidantien und/oder Komplexbildner zu formulieren, da diese die Wirkung der Enzyme blockieren können.

Die folgenden Beispiele sollen den Erfindungsgegenstand näher erläutern.

### Beispiel 1:

### a) Enzymlösung

Es wurde Phenoloxidase aus Stachybotrys bisbyi wie folgt hergestellt:
Die Anzucht des Stammes erfolgte in einer Zusammensetzung, die in Tabelle 1 aufgeführt ist. Diese Zusammensetzung wies einen pH-Wert von 5,4 auf und wurde autoklaviert.

**Tabelle 1:**

| Zusammensetzung für die Anzucht | |
|---|---|
| Komponente | Menge [g/l] |
| Glucose | 15 |
| N-Z-Soy-Peptone [Fa. Sigma] | 3 |
| KH₂PO₄ | 0,6 |
| ZnSO₄^{.} 7 H₂O | 0,001 |
| K₂HPO₄ | 0,04 |
| FeSO₄^{.} 7 H₂O | 0,005 |
| MnSO₄^{.} H₂O | 0,05 |
| MgSO₄^{.} 7 H₂O | 0,05 |
| CuSO₄^{.} 5 H₂O | 0,25 |
| Wasser | ad 1 l |

Dazu wurden 30 ml-Kulturen 14 Tage lang bei 150 rpm Schüttelfrequenz und 30 °C belassen. Nach 14 Tagen wurden die Kulturen bei 12 000 Umdrehungen/Minute abzentrifugiert.

Die Enzym-Aktivität des Kulturüberstandes wurde gemäß folgendem Test bestimmt. 1,5 ml wäßrige Natriumacetat-Lösung (120 mM/l, mit verdünnter Natronlauge auf pH 5 eingestellt) wurden mit 0,2 ml ABTS (2,2'-Azinobis(3-ethylbenzthiazolin-6-sulfonsäure) und 0,3 ml abzentrifugiertem Kulturüberstand versetzt. Die Umsetzung von ABTS durch die Enzym-Lösung wurde als Abnahme der Absorption bei 420 nm während eines Zeitraums von 2 Minuten im Spektralphotometer bestimmt. Die Aktivität der Enzymlösung wurde in ΔOD / min (OD= optische Dichte) ausgedrückt. Für die folgende Ausfärbung wurde ein Kulturüberstand mit einer Enzymaktivität von 8 [ΔOD/min] verwendet.

### b) Färbecreme:

Es wurde eine Basis-Creme der in Tabelle 2 aufgeführten Zusammensetzung hergestellt.

**Tabelle 2:**

| Basis-Creme: | |
|---|---|
| Komponente | Menge [g] |
| Lorol®, techn.¹ | 6,5 |
| Hydrenol® D² | 24,2 |
| Dehyton®K³ | 20,0 |
| Texapon®NSO⁴ | 32,0 |
| bidest. Wasser | ad 100 |

| | |
|---|---|
| ¹ C12-18-Fettalkohol (INCI-Bezeichnung: Coconut Alcohol) (COGNIS) | |
| ² C16-C18-Fettalkohol (INCI-Bezeichnung: Cetearyl alcohol) (COGNIS) | |
| ³ Fettsäureamid mit Betainstruktur (ca. 30% Aktivsubstanz; INCI-Bezeichnung: Cocamidopropyl Betaine) (Henkel) | |
| ⁴ Natriumsalz des Laurylethersulfats (ca. 27% Aktivsubstanz; INCI-Bezeichnung: Sodium Laureth Sulfate) (COGNIS) | |

12,5 g dieser Basis-Creme wurden mit bidest. Wasser auf 24 g aufgefüllt. Dann wurde der pH-Wert mit 1,5 %iger wäßriger Ammoniak-Lösung auf 7 eingestellt und die Creme durch Auffüllen mit bidest. Wasser auf 25 g fertiggestellt.

Zur Herstellung der Färbezubereitung wurde diese Creme unter ständigem Rühren erhitzt, bis sie flüssig war. Dann wurden die gelösten Entwickler- und Kupplersubstanzen (0,0025 mmol 4-Aminophenol in Wasser und 0,0025 mmol 5-Amino-2-methylphenol in Propylenglykol) zugegeben und die Creme bis zur Homogenität gerührt. Dabei kühlte die Creme auf ca. 30 °C ab. Für die Ausfärbungen wurden je 1 g dieser Färbezubereitung mit
- 1 g des Enzym-haltigen Kulturüberstandes (B1) oder
- 1 g einer 2%igen wäßrigen Wasserstoffperoxidlösung (V1) (Vergleichsversuch mit konventionellem Oxidationsmittel) oder
- 1 g bidest. Wasser (V2) (Vergleichsversuch ohne Oxidationsmittel) verrührt

Die 2 g dieser End-Zubereitung wurden auf eine Haarsträhne (Human-Haar naturweiß (Fa. Kerling), 0,5 g) aufgetragen. Nach einer Einwirkzeit von 30 Minuten bei Raumtemperatur wurde das Haar mit lauwarmem Wasser gespült und an der Luft getrocknet.

Die Beurteilung der erhaltenen Färbungen sind in Tabelle 3 zusammengestellt:

**Tabelle 3:**

| Färbeergebnisse | | | | |
|---|---|---|---|---|
| Versuch | Oxidationsmittel | Farbe¹ | ΔE-Wert² | Farbstärken-Änderung [%] im Vergleich zu V2 |
| B1 | Enzymlösung | persischorange | 19,35 | 244 |
| V1 | H₂O₂ | grauorange | 11,24 | 192 |
| V2 | - | blaßorange | 0 | 100 (Referenz) |

| | | | | |
|---|---|---|---|---|
| 1 A. Komerup und J.H. Wanscher, Taschenlexikon der Farben, Muster-Schmidt-Verlag, 3. Auflage 1981. | | | | |
| 2 Gemäß DIN 5033 (Teil 3) vom Juli 1992 Die Färbung der Strähnen wurde farbmetrisch an 4 Meßpunkten mit dem Gerät Datacolor Text Flash, der Firma Data Color International vermessen, die Meßergebnisse mit der Software Data Color Tools QC gemäß Formel (1) ausgewertet und in der folgenden Tabelle zusammengefaßt. Als Referenz diente die Färbung einer Strähne gemäß V2. | | | | |

$\frac{{\text{}}^{\text{K}} \text{/} {\text{}}_{{\text{S}}_{\text{Pr} \text{obe}}}}{{\text{}}^{\text{K}} \text{/} {\text{}}_{{\text{S}}_{\text{Re} \text{ferenz}}}} \text{* 100 =} \text{Farbstärke} \text{[%]}$ mit K = Absorptionskoeffizient
S = Streukoeffizient
K/S = Reflektionskoeffizient

Zusätzlich wurde gemäß Formel (2) der ΔE-Wert des CIELAB-Farbsystems ermittelt$\text{ΔE =} \sqrt{{\text{(ΔL)}}^{\text{2}} {\text{+ (ΔA)}}^{\text{2}} {\text{+ (ΔB)}}^{\text{2}}}$

### Beispiel 2:

Analog Beispiel 1 wurden weitere Ausfärbungen durchgeführt. Dabei wurden jeweils 8 g der in Tabelle 4 aufgeführten Färbezubereitungen 1 - 4 mit 2 g des Kulturüberstandes von Stachybotrys bisbyi mit einer Enzymaktivität von 8 [ΔOD/min] gemischt. Dann wurden 2 g dieser End-Zubereitung auf Human-Haar aufbetragen; das weitere Vorgehen erfolgte wie in Beispiel 1.

**Tabelle 4:**

| Komponente [g] | 1 | 2 | 3 | 4 |
|---|---|---|---|---|
| Lorol®, techn. | 3,2 | 3,2 | 3,2 | 3,2 |
| Eumulgin®B2⁵ | 0,5 | 0,5 | 0,5 | 0,5 |
| Hydrenol®D | 12,1 | 12,1 | 12,1 | 12,1 |
| Dehyton®K | 10,0 | 10,0 | 10,0 | 10,0 |
| Plantacare®1200⁶ | 3,0 | 3,0 | 3,0 | 3,0 |
| N-Acetyl-cystein | - | - | 0,5 | 1,0 |
| Natriumcitrat | 0,5 | 1,0 | - | - |
| 2,4,5,6-Tetraaminopyrimidinsulfat | 0,025 | 0,02057 | - | - |
| p-Toluylendiamin-sulfat | 0,025 | 0,8910 | 0,6144 | 0,96 |
| 3-Methyl-4-amino-phenol | 0,6 | 0,43 | 0,0518 | - |
| 4-Chlorresorcin | 0,0316 | 0,242 | 0,1753 | - |
| 2-Amino-3-hydroxypyridin | 0,5362 | - | - | - |
| 1-(2'-Hydroxyethyl)amino-4-methyl-2-nitrobenzol | 0,1 | - | - | - |
| 2-Methylresorcin | - | 0,242 | 0,0722 | - |
| 2,7-Dihydroxynaphthalin | - | 0,6 | - | - |
| m-Aminophenol | - | - | 0,0293 | 0,0893 |
| Resorcin | - | - | 0,1109 | - |
| 1,3-Bis-(2,4-diaminophenoxy)propan 4 HCl | - | - | - | 0,361 |
| 2-Methylamino-3-amino-5-methoxypyridin | - | - | - | 0,1853 |
| Isopropanolamin | | ad pH 7,0 | - | ad pH 7,0 |
| Monoethanolamin | ad pH 7,0 | - | ad pH 7,0 | - |
| bidest. Wasser | ←-------------------ad 100 g----------------------------------→ | | | |
| Ausfärbung | kupfergold | haselnuß | hellbraun | schwarz |

| | | | | |
|---|---|---|---|---|
| ⁵ Cetylstearylalkohol mit ca. 20 EO-Einheiten (INCI-Bezeichnung: Ceteareth-20) (COGNIS) | | | | |
| ⁶ C₁₂₋₁₆-Fettalkohol-1,4-glucosid (ca. 50 % Aktivsubstanz; INCI-Bezeichnung: Lauryl Glucoside)(COGNIS) | | | | |

### Beispiel 3:

Es wurden folgende Färbemittel hergestellt (alle Mengenangaben sind, soweit nicht anders vermerkt, Gewichtsteile).

| *1. Färbemittel A* | |
|---|---|
| Fettalkoholgemisch C₁₂-C₁₈ | 5,25 |
| Eumulgin® B2 | 0,4 |
| Plantacare® 1200 | 3,0 |
| Dehyton® K | 2,5 |
| Ammoniumsulfat | 0,5 |
| Natrosol® 250 HR⁷ | 1,0 |
| 4-Amino-2-((diethylamino)methyl)phenol- dihydrochlorid | 2,67 |
| 2-Amino-3-hydroxypyridin | 1,1 |
| Ammoniak (25-%ig in Wasser) | ad pH 8,5 |
| Wasser | ad 100 |

| | |
|---|---|
| ⁷ Hydroxyethylcellulose (Hercules) | |

| 2. *Färbemittel B* | |
|---|---|
| Fettalkoholgemisch C₁₂-C₁₈ | 5,25 |
| Eumulgin® B2 | 0,4 |
| Plantacare® 1200 | 3,0 |
| Dehyton® K | 2,5 |
| Ammoniumsulfat | 0,5 |
| Natrosol® 250 HR | 1,0 |
| 4-Amino-2-aminomethyl-phenol-dihydrochlorid | 0,002 |
| 2-Amino-3-hydroxypyridin | 0,004 |
| 3-Methyl-4-aminophenol | 0,007 |
| 1,3-Bis-(2,4-diarninophenoxy)-propan-tetrahydrochlorid | 0,0005 |
| p-Toluylendiamin-sulfat | 0,06 |
| Resorcin | 0,01 |
| 3-Amino-2,4-dichlorphenol-hydrochlorid | 0,008 |
| 3-Aminophenol | 0,002 |
| 4-Chlorresorcin | 0,01 |
| Ammoniak (25-%ig in Wasser) | ad pH 8,5 |
| Wasser | ad 100 |

| 3. *Färbemittel* C | |
|---|---|
| Fettalkoholgemisch C₁₂-C₁₈ | 5,25 |
| Eumulgin® B2 | 0,4 |
| Plantacare® 1200 | 3,0 |
| Dehyton® K | 2,5 |
| Ammoniumsulfat | 0,5 |
| Natrosol® 250 HR | 1,0 |
| 4-Amino-2-chlorphenol | 0,8 |
| 2-Amino-3-hydroxypyridin | 0,1 |
| 3-Methyl-4-aminophenol | 0,3 |
| 5-Amino-2-methylphenol | 0,07 |
| Resorcin | 0,2 |
| 3-Aminophenol | 0,2 |
| 1-(2'-Hydroxyethyl)-2,5-diaminobenzolsulfat | 0,7 |
| 4-Methylresorcin | 0,1 |
| 5,6-Dihydroxyindol | 0,05 |
| Monoethanolamin | ad pH 8,5 |
| Wasser | ad 100 |

| *4. Färbemittel D* | |
|---|---|
| Fettalkoholgemisch C₁₂-C₁₈ | 5,25 |
| Eumulgin® B2 | 0,4 |
| Plantacare® 1200 | 3,0 |
| Dehyton® K | 2,5 |
| Ammoniumsulfat | 0,5 |
| Natrosol® 250 HR | 1,0 |
| 4-Amino-phenol | 0,49 |
| 4-Amino-3-methylphenol | 0,55 |
| 2-Amino-3-hydroxypyridin | 1,0 |
| p-Toluylendiamin-sulfat | 0,2 |
| 6-Hydroxyindol | 0,1 |
| 5-((2'-Hydroxyethyl)-amino)-2-methylphenol | 0,1 |
| 1,2,3,4-Tetrahydro-6-nitrochinoxalin | 0,05 |
| HC Yellow 5⁸ | 0,03 |
| HC Red 1⁹ | 0,02 |
| Natriumhydroxid | ad pH 8,5 |
| Wasser | ad 100 |

| | |
|---|---|
| ⁸ 2-((2-Amino-4-nitrophenyl)amino)ethanol | |
| ⁹ 4-Amino-2-nitrodiphenylamin | |

| *5. Färbemittel E* | |
|---|---|
| Fettalkoholgemisch C₁₂-C₁₈ | 5,25 |
| Eumulgin® B2 | 0,4 |
| Plantacare® 1200 | 3,0 |
| Dehyton® K | 2,5 |
| Ammoniumsulfat | 0,5 |
| Natrosol® 250 HR | 1,0 |
| 4-Amino-2-aminomethyl-phenoldihydrochlorid | 2,0 |
| 2-Methylamino-3-amino-6-methoxypyridin | 2,25 |
| Arginin | ad pH 8,5 |
| Wasser | ad 100 |

| *6. Färbemittel F* | |
|---|---|
| Fettalkoholgemisch C₁₂-C₁₈ | 5,25 |
| Eumulgin® B2 | 0,4 |
| Plantacare® 1200 | 3,0 |
| Dehyton® K | 2,5 |
| Ammoniumsulfat | 0,5 |
| Natrosol® 250 HR | 1,0 |
| 4-Amino-2-aminomethyl-phenol-dihydrochlorid | 0,017 |
| 2-Methylamino-3-amino-6-methoxypyridin | 0,002 |
| 1-(2'-Hydroxyethyl)-2,5-diaminobenzolsulfat | 0,03 |
| 1-Naphthol | 0,02 |
| 2-Methylresorcin | 0,0006 |
| Lysin | ad pH 8,5 |
| Wasser | ad 100 |

| *7. Färbemittel G* | |
|---|---|
| Fettalkoholgemisch C₁₂-C₁₈ | 5,25 |
| Eumulgin® B2 | 0,4 |
| Plantacare® 1200 | 3,0 |
| Dehyton® K | 2,5 |
| Ammoniumsulfat | 0,5 |
| Natrosol® 250 HR | 1,0 |
| 4-Amino-2-((diethylamino)methyl)phenol-dihydrochlorid | 0,1 |
| 2-Methylamino-3-amino-6-methoxypyridin | 0,002 |
| 3-Methyl-4-aminophenol | 0,07 |
| 2,4,5,6-Tetraaminopyrimidin-sulfat | 1,0 |
| p-Toluylendiamin-sulfat | 1,1 |
| 3-Aminophenol | 0,006 |
| Resorcin | 0,11 |
| 2-Methylresorcin | 0,54 |
| 2,7-Dihydroxynaphthalin | 0,032 |
| 2-Amino-3-hydroxypyridin | 0,4 |
| 4-Amino-2-nitro-diphenylamin-2'-carbonsäure | 0,1 |
| Ammoniak, (25-%ig in Wasser) ad | pH 8,5 |
| Wasser | ad 100 |

| *8. Färbemittel* H | |
|---|---|
| Fettalkoholgemisch C₁₂-C₁₈ | 5,25 |
| Eumulgin® B2 | 0,4 |
| Plantacare® 1200 | 3,0 |
| Dehyton® K | 2,5 |
| Ammoniumsulfat | 0,5 |
| Natrosol® 250 HR | 1,0 |
| 4-Amino-2-chlorphenol-dihydrochlorid | 1,0 |
| 2-Methylamino-3-amino-6-methoxypyridin | 1,7 |
| 2-Amino-4-nitro-6-chlorphenol | 0,05 |
| HC Red BN¹⁰ | 0,2 |
| 5,6-Dihydroxyindolin-hydrobromid | 0,1 |
| Ammoniak (25-%ig in Wasser) | ad pH 8,5 |
| Wasser | ad 100 |

| | |
|---|---|
| ¹⁰ 4-(3-Hydroxypropyl)amino-3-nitrophenol (Clariant) | |

| *9*. *Färbemittel I* | |
|---|---|
| Fettalkoholgemisch C₁₂-C₁₈ | 5,25 |
| Eumulgin® B2 | 0,4 |
| Plantacare® 1200 | 3,0 |
| Dehyton® K | 2,5 |
| Ammoniumsulfat | 0,5 |
| Natrosol® 250 HR | 1,0 |
| 4-Amino-2-((diethylamino)methyl)phenol-dihydrochlorid | 1,25 |
| 4-Amino-2-aminomethyl-phenol-dihydrochlorid | 1,0 |
| 5-Amino-2-methyl-phenol | 1,23 |
| Ammoniak (25-%ig in Wasser) | ad pH 8,5 |
| Wasser | ad 100 |

| *10. Färbemittel J* | |
|---|---|
| Fettalkoholgemisch C₁₂-C₁₈ | 5,25 |
| Eumulgin® B2 | 0,4 |
| Plantacare® 1200 | 3,0 |
| Dehyton® K | 2,5 |
| Ammoniumsulfat | 0,5 |
| Natrosol® 250 HR | 1,0 |
| 4-Amino-2-((diethylamino)methyl)phenol-dihydrochlorid | 0,5 |
| Bis-(2-hydroxy-5-aminophenyl)methan | 0,4 |
| 5-Amino-2-methylphenol | 0,55 |
| p-Toluylendiamin-sulfat | 0,3 |
| p-Phenylendiamin-dihydrochlorid | 0,2 |
| Resorcin | 0,07 |
| 2,7-Dihydroxynaphthalin | 0,09 |
| 2-Methylresorcin | 0,07 |
| o-Aminophenol | 0,03 |
| 5,6-Dihydroxyindolin-hydrobromid | 0,05 |
| Ammoniak (25-%ig in Wasser) | ad pH 8,5 |
| Wasser | ad 100 |

| *11. Färbemittel K* | |
|---|---|
| Fettalkoholgemisch C₁₂-C₁₈ | 5,25 |
| Eumulgin® B2 | 0,4 |
| Plantacare® 1200 | 3,0 |
| Dehyton® K | 2,5 |
| Ammoniumsulfat | 0,5 |
| Natrosol® 250 HR | 1,0 |
| 4-Amino-2-aminomethyl-phenol-dihydrochlorid | 0,02 |
| 5-Amino-2-methylphenol | 0,03 |
| 3-Methyl-4-aminophenol | 0,01 |
| 2,4,5,6-Tetraaminopyrimidin-sulfat | 0,8 |
| p-Toluylendiamin-sulfat | 0,09 |
| 3-Aminophenol | 0,004 |
| Resorcin | 0,04 |
| 2-Methylresorcin | 0,22 |
| 2,7-Dihydroxynaphthalin | 0,3 |
| 4-Amino-2-nitro-diphenylamin-2'-carbonsäure | 0,15 |
| 1,2,3,4-Tetrahydro-6-nitrochinoxalin | 0,25 |
| Ammoniak (25-%ig in Wasser) | ad pH 8,5 |
| Wasser | ad 100 |

| *12*. *Färbemittel L* | |
|---|---|
| Fettalkoholgemisch C₁₂-C₁₈ | 5,25 |
| Eumulgin® B2 | 0,4 |
| Plantacare® 1200 | 3,0 |
| Dehyton® K | 2,5 |
| Natriumsulfit | 0,1 |
| Ammoniumsulfat | 0,5 |
| Natrosol® 250 HR | 1,0 |
| 4-Amino-2-aminomethyl-phenol-dihydrochlorid | 1,06 |
| 4-Aminophenol | 0,55 |
| 5-Amino-2-methylphenol | 0,74 |
| 4-Hydroxyindol | 0,1 |
| 1,3-Bis-(2,4-diaminophenoxy)-propan-tetrahydrochlorid | 0,3 |
| 2-Methylamino-3-amino-6-methoxy-pyridin-dihydrochlorid | 0,1 |
| Ammoniak (25-%ig in Wasser) | ad pH 8,5 |
| Wasser | ad 100 |

| *13. Färbemittel M* | |
|---|---|
| Hydrenol®D | 8,0 |
| C12-C18-Fettalkohol | 2,0 |
| Akypo Soft® 45 NV¹¹ | 10,0 |
| Eumulgin®B1¹² | 0,5 |
| Eumulgin®B2 | 0,5 |
| 5-Amino-2-methylphenol | 0,02 |
| 3-Amino-2-chlor-6-methylphenol | 0,36 |
| Bis-(2,4-diaminophenoxy)propan-tetrahydrochlorid | 0,0002 |
| 3-Methyl-4-aminophenol | 0,02 |
| 4-Amino-2-aminomethyl-phenol-dihydrochlorid | 0,40 |
| p-Toluylendiamin-sulfat | 0,34 |
| N,N-Bis-(2-hydroxyethyl)-p-phenylendiamin-Sulfat | 0,48 |
| Resorcin | 0,33 |
| m-Aminophenol | 0,001 |
| 2-Amino-3-hydroxypyridin | 0,009 |
| Ammoniumchlorid | 0,3 |
| Ammoniak (25%ig in Wasser) ad pH | 8,5 |
| Wasser | ad 100 |

| | |
|---|---|
| ¹¹ C12-14-Fettalkohol-4,5-EO-essigsäure-Natrium-Salz (21 % Aktivsubstanz in Wasser; INCI-Bezeichnung: Sodium Laureth-6 Carboxylate) (KAO) | |
| ¹² Cetylstearylalkohol + 12 EO (INCI-Bezeichnung: Ceteareth-12) (COGNIS) | |

| *14. Färbemittel N* | |
|---|---|
| Hydrenol®D | 8,0 |
| C12-C18-Fettalkohol | 2,0 |
| Akypo Soft® 45 NV | 10,0 |
| Eumulgin®B1 | 0,5 |
| Eumulgin®B2 | 0,5 |
| m-Aminophenol | 0,005 |
| 3-Amino-2-methylamino-6-methoxypyridin-hydrochlorid | 0,002 |
| 2-Amino-3-hydroxypyridin | 0,24 |
| 2,7-Dihydroxynaphthalin | 0,02 |
| 2-Methylresorcin | 0,6 |
| p-Toluylendiamin-sulfat | 0,73 |
| Resorcin | 0,13 |
| 2,6-Dihydroxy-3,4-dimethyl-pyridin | 0,1 |
| 2,4,5,6-Tetraaminopyrimidin | 1,3 |
| 5-Amino-2-methylphenol | 0,03 |
| 5-(β-Hydroxyethyl)amino-2-methylphenol | 0,3 |
| 2-Nitro-4-aminodiphenylamin-2'-carbonsäure | 0,1 |
| Ammoniumchlorid | 0,4 |
| Parfümöl | 0,2 |
| Ammoniak (25%ig in Wasser) | ad pH 8,5 |
| Wasser | ad 100 |

| *15. Färbemittel O* | |
|---|---|
| Hydrenol®D | 8,0 |
| C12-C18-Fettalkohol | 2,0 |
| Akypo Soft® 45 NV | 10,0 |
| Eumulgin®B1 | 0,5 |
| Eumulgin®B2 | 0,5 |
| 2-Methylresorcin | 0,48 |
| p-Toluylendiamin-sulfat | 0,6 |
| 1-(2'-Hydroxyethyl)-2,5-diaminobenzol | 0,6 |
| Resorcin | 0,21 |
| 2,6-Dihydroxy-3,4-dimethyl-pyridin | 0,66 |
| 2,4,5,6-Tetraaminopyrimidin | 3,0 |
| 3-Methyl-4-aminophenol | 0,16 |
| 3-Am ino-2-chlor-6-methyl-phenol | 0,11 |
| 5-Amino-2-methylphenol | 0,03 |
| 1,5-Diyhdroxynaphthalin | 0,002 |
| Ammoniumchlorid | 0,2 |
| Mirapol®A-15¹³ | 1,5 |
| Parfümöl | 0,2 |
| Histidin | ad pH 8,5 |
| Wasser | ad 100 |

| | |
|---|---|
| ¹³ polymeres quaternäres Ammoniumsalz (ca. 64 % Aktivsubstanz; INCI-Bezeichnung: Polyquatemium-2) (RHONE-POULENC) | |

| *16. Färbemittel P* | |
|---|---|
| Hydrenol®D | 5,0 |
| C12-C18-Fettalkohol | 2,0 |
| Texapon®NSO | 3,0 |
| Ammoniumsulfat | 0,5 |
| 5-Amino-2-methylphenol | 0,56 |
| 3-Methyl-4-aminophenol | 0,055 |
| p-Toluylendiamin-sulfat | 0,65 |
| Resorcin | 0,14 |
| 4-Aminophenol-hydrochlorid | 0,6 |
| 4-Amino-2-nitro-diphenylamino-2'-carbonsäure | 0,05 |
| 6-Nitro-1,2,3,4-tetrahydrochinoxalin | 0,1 |
| Ammoniak (25%ig in Wasser) | ad pH 8,5 |
| Wasser | ad 100 |

| *17. Färbemittel Q* | |
|---|---|
| Hydrenol®D | 5,0 |
| C 12-C18-Fettalkohol | 2,0 |
| Texapon®NSO | 3,0 |
| Ammoniumsulfat | 0,5 |
| Bis-(2,4-diaminophenoxy)propan-tetrahydrochlorid | 0,003 |
| p-Toluylendiamin-sulfat | 0,033 |
| Resorcin | 0,0055 |
| 4-Aminophenol-hydrochlorid | 0,014 |
| 2-Amino-3-hydroxypyridin | 0,0035 |
| Ammoniak (25%ig in Wasser) | ad pH 8,5 |
| Wasser | ad 100 |

Zur Ausfärbung wurde jeweils 1 Teil der oben beschriebenen Färbemittel mit 0,1 Teil einer Enzymzubereitung vermischt. Die Enzymzubereitung enthielt, bezogen auf die Proteinmenge, 0,3 Gew.-% Enzym, gewonnen aus Stachybotrys parvispora bzw. Stachybotrys chartarum. ,

Diese Anwendungszubereitung wurde auf zu 80 % ergrautes mittelblondes Menschenhaar aufgebracht, dort für 30 Minuten belassen und dann ausgespült. Die Ergebnisse dieser Ausfärbungen, wobei bei Verwendung der beiden genannten Enzyme hinsichtlich der Farbrichtung vergleichbare Ausfärbungen erhalten wurden, sind in der folgenden Tabelle zusammengestellt

| | |
|---|---|
| Färbemittel | Ausfärbung |
| A | möhrenrot |
| B | birkengrau |
| C | lederbraun |
| D | rotbraun |
| E | rötlichbraun |
| F | birkengrau |
| G | portweinrot |
| H | achatbraun |
| I | blaßorange |
| J | violettbraun |
| K | englischrot |
| L | madeirabraun |
| M | goldbraun |
| N | rubinrot |
| O | granat |
| P | mahagoni |
| Q | lichtaschblond |

## Patentansprüche

1. Mittel zum Färben keratinischer Fasern enthaltend in einem kosmetisch akzeptablen Träger mindestens ein Farbstoffvorprodukt, **dadurch gekennzeichnet, daß** es mindestens ein Phenol-oxidierendes Enzym, das aus *Stachybotrys Spezies* gewonnen werden kann, enthält.

2. Mittel nach Anspruch 1, **dadurch gekennzeichnet, daß** das Enzym gereinigt ist.

3. Mittel nach einem der Ansprüche 1 oder 2, **dadurch gekennzeichnet, daß** das Enzym aus *Stachybotrys parvispora*, *Stachybotrys chartarum*, *Stachybotrys kampalensis*, *Stachybotrys theobromae*, *Stachybotrys bisbyi*, *Stachybotrys cylindrospora*, *Stachybotrys dichroa*, *Stachybotrys oenanthes* und *Stachybotrys nilagerica* ausgewählt ist.

4. Mittel nach einem der Ansprüche 1 bis 3, **dadurch gekennzeichnet, daß** es als Farbstoffvorprodukt mindestens eine Entwicklerkomponente enthält.

5. Mittel nach einem der Ansprüche 1 bis 4, **dadurch gekennzeichnet, daß** es als Farbstoffvorprodukt mindestens ein Indol und/oder Indolinderivat enthält.

6. Mittel nach einem der Ansprüche 1 bis 5, **dadurch gekennzeichnet, daß** es mindestens eine Kupplerkomponente enthält.

7. Mittel nach einem der Ansprüche 1 bis 6, **dadurch gekennzeichnet, daß** es weiterhin mindestens einen direktziehenden Farbstoff enthält.

8. Mittel nach einem der Ansprüche 1 bis 7, **dadurch gekennzeichnet, daß** es weiterhin mindestens ein Tensid enthält.

9. Mittel nach Anspruch 8, **dadurch gekennzeichnet, daß** es ein nichtionisches oder ein amphoteres Tensid enthält.

10. Mittel nach einem der Ansprüche 8 oder 9, **dadurch gekennzeichnet, daß** es eine Tensidkombination aus mindestens einem anionischen Tensid mit mindestens einem amphoteren Tensid und/oder mit mindestens einem nichtionischen Tensid enthält.

11. Mittel nach einem der Ansprüche 1 bis 10, **dadurch gekennzeichnet, daß** es ein Alkalisierungsmittel enthält

12. Verfahren zum Färben keratinischer Fasern, **dadurch gekennzeichnet, daß** ein Mittel nach einem der Ansprüche 1 bis 11 eingesetzt wird.

13. Verwendung eines Phenol-oxidierenden Enzyms, das aus *Stachybotrys Spezies* gewonnen werden kann, zur oxidativen Färbung keratinischer Fasern.

## Claims

1. A composition for colouring keratinous fibres containing at least one dye precursor in a cosmetically acceptable carrier, **characterized in that** it contains at least one phenol-oxidizing enzyme obtainable from *Stachybotrys* species.

2. A compositions as claimed in claim 1, **characterized in that** the enzyme is purified.

3. A composition as claimed in claim 1 or 2, **characterized in that** the enzyme is selected from *Stachybotrys parvispora, Stachybotrys chartarum, Stachybotrys kampalensis, Stachybotrys theobromae, Stachybotrys bisbyi, Stachybotrys cylindrospora, Stachybotrys dichroa, Stachybotrys oenanthes and Stachybotrys nilagerica*.

4. A composition as claimed in any of claims 1 to 3 **characterized in that** it contains at least one primary intermediate as the dye precursor.

5. A composition as claimed in any of claims 1 to 4, **characterized in that** it contains at least one indole and/or indoline derivative as the dye precursor.

6. A composition as claimed in any of claims 1 to 5, **characterized in that** it contains at least one secondary intermediate.

7. A composition as claimed in any of claims 1 to 6, **characterized in that** it also contains at least one substantive dye.

8. A composition as claimed in any of claims 1 to 7, **characterized in that** it also contains at least one surfactant.

9. A composition as claimed in claim 8, **characterized in that** it contains a nonionic or amphoteric surfactant.

10. A composition as claimed in claim 8 or 9, **characterized in that** it contains a surfactant combination of at least one anionic surfactant with at least one amphoteric surfactant and/or with at least one nonionic surfactant.

11. A composition as claimed in any of claims 1 to 10, **characterized in that** it contains an alkalizing agent.

12. A process for colouring keratinous fibres, **characterized in that** the composition claimed in any of claims 1 to 11 is used.

13. The use of a phenol-oxidizing enzyme obtainable from *Stachybotrys* species for the oxidative colouring of keratinous fibres.

## Revendications

1. Agent pour la coloration de fibres kératiniques contenant, dans un support acceptable du point de vue cosmétique, au moins un précurseur de colorant, **caractérisé en ce qu'**il contient au moins une enzyme d'oxydation à base de phénol, que l'on peut obtenir à partir de *Stachybotrys Spezies.*

2. Agent selon la revendication 1, **caractérisé en ce que** l'enzyme est purifiée.

3. Agent selon l'une quelconque des revendications 1 ou 2, **caractérisé en ce que** l'enzyme est sélectionnée à partir du groupe comprenant *Stachybotrys parvispora, Stachybotrys chartarum, Stachybotrys kampalensis, Stachybotrys theobromae, Stachybotrys bisbyi, Stachybotrys cylindrospora, Stachybotrys dichroa, Stachybotrys oenanthes* et *Stachybotrys nilagerica*.

4. Agent selon l'une quelconque des revendications 1 à 3, **caractérisé en ce qu'**il contient, à titre de précurseur de colorant, au moins un composant faisant office de révélateur.

5. Agent selon l'une quelconque des revendications 1 à 4, **caractérisé en ce qu'**il contient, à titre de précurseur de colorant, au moins un indole et/ou un dérivé d'indoline.

6. Agent selon l'une quelconque des revendications 1 à 5, **caractérisé en ce qu'**il contient au moins un composant faisant office de copulant.

7. Agent selon l'une quelconque des revendications 1 à 6, **caractérisé en ce qu'**il contient, en outre, au moins un colorant montant directement sur la fibre.

8. Agent selon l'une quelconque des revendications 1 à 7, **caractérisé en ce qu'**il contient, en outre, au moins un agent tensioactif.

9. Agent selon la revendication 8, **caractérisé en ce qu'**il contient un agent tensioactif non ionique ou un agent tensioactif amphotère.

10. Agent selon l'une quelconque des revendications 8 ou 9, **caractérisé en ce qu'**il contient une combinaison d'agent tensioactif constitué par au moins un agent tensioactif anionique avec au moins un agent tensioactif amphotère et/ou avec au moins un agent tensioactif non ionique.

11. Agent selon l'une quelconque des revendications 1 à 10, **caractérisé en ce qu'**il contient un agent rendant alcalin.

12. Procédé pour la coloration de fibres kératiniques, **caractérisé en ce qu'**on met en oeuvre un agent selon l'une quelconque des revendications 1 à 11.

13. Utilisation d'une enzyme d'oxydation à base de phénol, que l'on peut obtenir à partir de *Stachybotrys Spezies,* pour la coloration de fibres kératiniques par oxydation.
